# EUROPEAN PATENT SPECIFICATION

(11) **EP 3 393 559 B1**
(45) Date of publication and mention of the grant of the patent: **09.03.2022**
(21) Application number: 16809733.5
(22) Date of filing: 06.12.2016
(51) Int. Cl.: A24F 40/42, A24F 40/48, A61M 15/06, A61M 15/00, A61M 11/00, A61M 11/04

(54) **AEROSOL-GENERATING SYSTEM WITH MOTOR**
AEROSOLERZEUGENDES SYSTEM MIT MOTOR
SYSTÈME DE GÉNÉRATION D'AÉROSOL AVEC MOTEUR

(30) Priority: 22.12.2015 EP 15202139
(43) Date of publication of application: 31.10.2018
(73) Proprietor: Philip Morris Products S.A., 2000 Neuchâtel (CH)
(72) Inventor: BATISTA, Rui Nuno, 1110 Morges (CH); MAZUR, Ben, Bristol BS7 9QP (GB)
(74) Representative: Ponder, William Anthony John
(86) International application number: PCT/EP2016/079944
(87) International publication number: WO 2017/108394

(56) References cited:
- EP-A1- 2 047 880
- US-A- 5 743 251
- US-A1- 2015 216 237

## Description

The present invention relates to aerosol-generating systems, such as handheld electrically operated smoking systems. In particular, the present invention relates to aerosol-generating systems in which the aerosol-forming substrate is liquid and is contained in a liquid storage portion.

One type of aerosol-generating system is an electrically operated smoking system. Handheld electrically operated smoking systems are known that consist of a device portion comprising a battery and control electronics, a cartridge portion comprising a supply of aerosol-forming substrate held in a liquid storage portion, and an electrically operated vaporiser. A cartridge comprising both a supply of aerosol-forming substrate held in the liquid storage portion and a vaporiser is sometimes referred to as a "cartomiser". The vaporiser typically comprises a coil of heater wire wound around an elongate wick soaked in the liquid aerosol-forming substrate held in the liquid storage portion. The cartridge portion typically comprises not only the supply of aerosol-forming substrate and an electrically operated vaporiser, but also a mouthpiece, which the user sucks on in use to draw aerosol into their mouth.

EP 2 047 880 A1 discloses an aerosol aspirator having a casing with a mouthpiece and an outside air inlet, a generation passage extending from the outside air inlet to the mouthpiece and a syringe pump. The syringe pump is arranged within the casing to deliver a solution to a distributing position in the generation passage in a fixed amount each time it is activated. A tubular heater is disposed downstream of the distributing position and forms a part of the generation passage.

EP 0 957 959 B1 discloses an electrically operated aerosol generator for receiving liquid material from a source, the aerosol generator comprising a pump for pumping the liquid material in metered amounts from the source through a tube with an open end, and a heater surrounding the tube. When heating the liquid material by the heater, the volatized material expands by exiting the open end of the tube.

Residues are created upon heating. In capillary tubes, the residues can cause clogging. This effect can alter liquid transport properties. Furthermore, the liquid material is heated indirectly: First the tube or a capillary wick is heated which in turn heats the liquid material. Heat can therefore be lost during the energy transfer process.

It would be desirable to provide an improved aerosol-generating system with a low-maintenance liquid transport system and reduced power consumption.

The invention is defined in the appended independent claim. Advantageous features are set out in the dependent claims.

According to a first aspect there is provided an aerosol-generating system comprising: a main unit, a cartridge removably coupled to the main unit, a power supply provided in the main unit, a liquid storage portion provided in the cartridge, for storing liquid aerosol-forming substrate, wherein the liquid storage portion comprises a movable wall and an outlet, a vaporiser comprising a heating element having a structure defining an open-ended internal passage, a pump that delivers liquid aerosol-forming substrate from the outlet of the liquid storage portion to the internal passage of the heating element, where it is heated to a temperature sufficient to volatilize at least a part of the delivered liquid aerosol-forming substrate, the pump comprising a micro stepper motor provided in the main unit, with a drive shaft that is configured to rotate for a predetermined amount upon performing one step of the micro stepper motor, a piston connected to the movable wall, and a lead screw connecting the drive shaft to the piston and configured to translate a rotation of the drive shaft into an axial movement of the piston and a corresponding axial movement of the movable wall, wherein the vaporiser heats the delivered liquid aerosol-forming substrate in the internal passage to a temperature sufficient to volatilize at least a part of the delivered liquid aerosol-forming substrate.

A determined amount of liquid aerosol-forming substrate may be pumped from the liquid storage portion to the internal passage of the heating element. By depositing the liquid aerosol-forming substrate to the heating element directly, the liquid aerosol-forming substrate can remain in its liquid state until it reaches the heating element. Consequently, few residues might be produced during liquid transport. Such a design can allow for production of cartridges without vaporisers. Due to the improved liquid transport, tubing segments and vaporisers might not need to be disposed once the liquid storage portion is empty. By using a pump instead of a capillary wick or any other passive medium to draw liquid, only the actually required amount of liquid aerosol-forming substrate may be transported to the heating element. Liquid aerosol-forming substrate may only be pumped upon demand, for example a request for a puff.

The implementation of the pump by a micro stepper motor and a lead screw may permit miniaturization as compared to prior micro pump designs. As the liquid aerosol-forming substrate may never have to enter and exit the pump, a number of potential failure modes like clogging or priming of the pump might be eliminated. Furthermore, as compared to piezo micro pump designs, the programming of the micro stepper motor may be far less complex so that simpler electronic circuitry might be required.

In contrast to micro pump designs, backflow of the pumped liquid aerosol-forming substrate may be eliminated, for example unless the micro stepper motor is operated in reverse mode for actively pulling back liquid aerosol-forming substrate.

The micro stepper motor may allow on-demand delivery of liquid aerosol-forming substrate for example at a low flow rate of approximately 0.5 to 2 microlitres per second for intervals of variable or constant duration. The micro stepper motor can be carefully tuned to precisely actuate the piston for a determined micro distance in order to deliver the appropriate amount of liquid aerosol-forming substrate to the heating element. The amount of liquid aerosol-forming substrate pumped by the micro stepper motor can be precisely adjusted, as the movement of the piston is based on the pitch of the turning lead screw. Consequently, the amount of deposited liquid aerosol-forming substrate can be determined from the amount of micro stepper motor pulses.

Both the micro stepper motor and the heating element may be triggered by a puff detection system. In some examples, the micro stepper motor and the heating element may be triggered by pressing an on-off button, held for the duration of a puff.

The micro stepper motor may step less than 1 degree per pulse. Assuming a rotation of 1 degree, a pitch on the thread of 0.75 millimetre and a capsule with a cross-section of 6 mm², liquid aerosol-forming substrate may be dispensed in increments of 0.0125 mm³ (0.0125 µl).

Preferably, the liquid storage portion is configured such that the axial movement of the movable wall towards the liquid storage portion causes a reduction of the volume of the liquid storage portion for example so as to deliver a determined amount of liquid aerosol-forming from the outlet of the liquid storage portion to the internal passage of the heating element upon performing one step of the micro stepper motor.

Preferably, the micro stepper motor is further configured to perform a step in reverse direction, thereby increasing the volume of the liquid storage portion. Reversing between puffs may be advantageous because liquid aerosol-forming substrate located in the transport system is reversed back into the liquid storage portion.

Preferably, the movable wall is configured to contain the liquid aerosol-forming substrate in the liquid storage portion for example so that the micro stepper motor and the piston are not in contact with the liquid aerosol-forming substrate. The liquid storage portion may comprise a syringe with a capsule, wherein the liquid aerosol-forming substrate is stored within the volume of the capsule that is limited by the movable wall. The capsule may have a cylindrical shape.

Preferably, the liquid storage portion is separated from the micro stepper motor, thereby having the possibility of a removable and throw-away liquid containing capsule. This would eradicate the need for the users to refill the liquid storage portion themselves.

Preferably, the aerosol-generating system further comprises a chamber into which the liquid aerosol-forming substrate is delivered, and wherein the heating element is arranged inside the chamber downstream of the outlet of the liquid storage portion.

As used herein, the terms 'upstream', 'downstream', 'proximal', 'distal', 'front' and 'rear', are used to describe the relative positions of components, or portions of components, of the aerosol-generating system in relation to the direction in which a user draws on the aerosol-generating system during use thereof.

The aerosol-generating system may comprise a mouth end through which in use an aerosol exits the aerosol-generating system and is delivered to a user. The mouth end may also be referred to as the proximal end. In use, a user draws on the proximal or mouth end of the aerosol-generating system in order to inhale an aerosol generated by the aerosol-generating system. The aerosol-generating system comprises a distal end opposed to the proximal or mouth end. The proximal or mouth end of the aerosol-generating system may also be referred to as the downstream end and the distal end of the aerosol-generating system may also be referred to as the upstream end. Components, or portions of components, of the aerosol-generating system may be described as being upstream or downstream of one another based on their relative positions between the proximal, downstream or mouth end and the distal or upstream end of the aerosol-generating system.

Preferably, the aerosol-generating system further comprises a tubing segment through which the liquid aerosol-forming substrate is delivered from the liquid storage portion to the vaporiser. The tubing segment may be arranged to deliver the liquid aerosol-forming substrate directly to the heating element. The tubing segment may be arranged to deliver the liquid aerosol-forming substrate towards an open end of the internal passage in the heating element. The tubing segment may extend from the liquid storage portion in a direction towards an open end of the internal passage in the heating element. The vaporiser may be arranged downstream of an open end of the tubing segment. The vaporiser may extend around a portion of the tubing segment.

The tubing segment, also referred to as tube, may be a nozzle. The tubing segment may comprise any appropriate material, for example glass, silicon, metal, for example stainless steel, or plastics material, for example PEEK. For example, the tube may have a diameter of about 1 to 2 millimetres but other sizes are possible. Preferably, the tubing segment comprises a capillary tube. The cross-section of the capillary tube may be circular, ellipsoid, triangular, rectangular or any other suitable shape to convey liquid. At least a width dimension of the cross-sectional area of the capillary tube is preferably chosen to be sufficiently small such that on the one hand capillary forces are present. At the same time, the cross-sectional area of the capillary tube is preferably sufficiently large such that a suitable amount of liquid aerosol-forming substrate can be conveyed to the heating element. In general, the cross-sectional area of the capillary tube is preferably below 4 square millimetres, below 1 square millimetre, or below 0.5 square millimetres.

The vaporiser may comprise a heating coil extending from the tubing segment in longitudinal direction. Alternatively, or in addition, the heating element, which may be a coil, may extend around a portion of the tubing segment. In some examples, the heating coil may be mounted transverse to the tubing segment. The heating coil may overlap with the open end of the tubing segment for up to 3 millimetres, preferably for up to 1 millimetre. In some examples, there may be a distance between the open end of the tubing segment and the heating coil. The length of the heating coil may be 2 millimetres to 9 millimetres, preferably 3 millimetres to 6 millimetres. The diameter of the heating coil may be chosen such that one end of the heating coil can be mounted around the tubing segment. The diameter of the heating coil may be 1 millimetre to 5 millimetres, preferably 2 millimetres to 4 millimetres.

The vaporiser may comprise a conical heater extending from the tubing segment in longitudinal direction. The conical heater may overlap with the open end of the tubing segment. In some examples, there may be a distance of 0.1 millimetres to 2 millimetres between the open end of the tubing segment and the conical heater, preferably 0.1 millimetres to 1 millimetre. The slant height of the conical heater may be 2 millimetres to 7 millimetres, preferably 2.5 millimetres to 5 millimetres. The diameter of the conical heater in cross-sectional view increases, when following the slant height from one end to the other, from a first diameter to a second diameter. The first diameter may be 0.1 millimetres to 2 millimetres, preferably 0.1 millimetres to 1 millimetre. The second diameter may be 1.2 millimetres to 3 millimetres, preferably 1.5 millimetres to 2 millimetres. Preferably, the conical heater is arranged such that the liquid aerosol-forming substrate exiting from the tubing segment passes the conical heater at the first diameter before the second diameter. The first diameter of the conical heater may be chosen such that one end of the conical heater can be mounted around the tubing segment.

The vaporiser may comprise a solid or mesh surface. The vaporiser may comprise a mesh heater. The vaporiser may comprise an arrangement of filaments.

The vaporiser may comprise at least one of a solid, flexible, porous, and perforated substrate onto which the heating element may be at least one of mounted, printed, deposited, etched, and laminated. The substrate may be a polymeric or ceramic substrate.

Preferably, the liquid storage portion comprises a one-way valve connected to the outlet of the liquid storage portion.

Preferably, the flow rate of the liquid aerosol-forming substrate delivered through the outlet of the liquid storage portion is within 0.5 to 2 microlitres per second.

Preferably, the main unit further comprises the vaporiser. The main unit may comprise a tubing segment.

The aerosol-generating system according to an embodiment of the present invention may further comprise electric circuitry connected to the vaporiser and to an electrical power source, the electric circuitry configured to monitor the electrical resistance of the vaporiser, and to control the supply of power to the vaporiser dependent on the electrical resistance of the vaporiser.

The electric circuitry may comprise a controller with a microprocessor, which may be a programmable microprocessor. The electric circuitry may comprise further electronic components. The electric circuitry may be configured to regulate a supply of power to the vaporiser. Power may be supplied to the vaporiser continuously following activation of the system or may be supplied intermittently, such as on a puff-by-puff basis. The power may be supplied to the vaporiser in the form of pulses of electrical current.

The aerosol-generating system advantageously comprises a power supply, typically a battery, within the main body of the housing. In some examples, the power supply may be another form of charge storage device such as a capacitor. The power supply may require recharging and may have a capacity that allows for the storage of enough energy for one or more smoking experiences; for example, the power supply may have sufficient capacity to allow for the continuous generation of aerosol for a period of around six minutes or for a period that is a multiple of six minutes. In some examples, the power supply may have sufficient capacity to allow for a predetermined number of puffs or discrete activations of the heater assembly.

For allowing ambient air to enter the aerosol-generating system, a wall of the housing of the aerosol-generating system, preferably a wall opposite the vaporiser, preferably a bottom wall, is provided with at least one semi-open inlet. The semi-open inlet allows air to enter the aerosol-generating system, but no air or liquid to leave the aerosol-generating system through the semi-open inlet. A semi-open inlet may for example be a semi-permeable membrane, permeable in one direction only for air, but is air- and liquid-tight in the opposite direction. A semi-open inlet may for example also be a one-way valve. Preferably, the semi-open inlets allow air to pass through the inlet only if specific conditions are met, for example a minimum depression in the aerosol-generating system or a volume of air passing through the valve or membrane.

The liquid aerosol-forming substrate is a substrate capable of releasing volatile compounds that can form an aerosol. The volatile compounds may be released by heating the liquid aerosol-forming substrate. The liquid aerosol-forming substrate may comprise plant-based material. The liquid aerosol-forming substrate may comprise tobacco. The liquid aerosol-forming substrate may comprise a tobacco-containing material containing volatile tobacco flavour compounds, which are released from the liquid aerosol-forming substrate upon heating. The liquid aerosol-forming substrate may alternatively comprise a non-tobacco-containing material. The liquid aerosol-forming substrate may comprise homogenised plant-based material. The liquid aerosol-forming substrate may comprise homogenised tobacco material. The liquid aerosol-forming substrate may comprise at least one aerosol-former. The liquid aerosol-forming substrate may comprise other additives and ingredients, such as flavourants.

The aerosol-generating system may be an electrically operated smoking system. Preferably, the aerosol-generating system is portable. The aerosol-generating system may have a size comparable to a conventional cigar or cigarette. The smoking system may have a total length between approximately 30 millimetres and approximately 150 millimetres. The smoking system may have an external diameter between approximately 5 millimetres and approximately 30 millimetres.

According to a second aspect of this disclosure the cartridge comprises the piston, and the lead screw. The lead screw comprises an opening that is configured to receive the drive shaft of the micro stepper motor. Preferably, the outlet of the liquid storage portion is configured to receive a tubing segment through which liquid aerosol-forming substrate is delivered to the deposition region of the heating element.

Preferably, the cartridge comprises a first cover that covers at least one of the movable wall of the liquid storage portion, the piston, and the lead screw before inserting the cartridge into the main unit. The first cover may be a pulled sticker or a seal, for example a film seal, to protect the cartridge before use, so that the movable wall cannot be accidently pushed before insertion into the main unit. The first cover could be removed from the cartridge by hand before inserting the cartridge into the main unit. Preferably, the first cover is punctured or pierced so that the first cover opens automatically upon inserting the cartridge into the main unit.

Preferably, the cartridge further comprises a second cover that covers the outlet of the liquid storage portion before inserting the cartridge into the main unit. The second cover may be a pulled sticker or a seal, for example a film seal, to protect the cartridge before use, so that the outlet cannot be accidently damaged before insertion of the cartridge into the main unit. The second cover could be removed from the cartridge by hand before inserting the cartridge into the main unit. Preferably, the second cover is punctured or pierced so that the second cover opens automatically upon inserting the cartridge into the main unit.

The cartridge may be a disposable article to be replaced with a new cartridge once the liquid storage portion of the cartridge is empty or below a minimum volume threshold. Preferably, the cartridge is pre-loaded with liquid aerosol-forming substrate. The cartridge may be refillable.

The cartridge and its components, including the lead screw, the piston, and the movable wall, may be made of thermoplastic polymers, such as polyether ether ketone (PEEK).

Embodiments of the invention will now be described, by way of example only, with reference to the accompanying drawings, in which:
Figure 1A is a topside view of an example for an aerosol-generating system;
Figure 1B is a topside view of an aerosol-generating system in accordance with an embodiment of the present invention;
Figure 1C is a topside view of an aerosol-generating system in accordance an embodiment of the present invention;
Figure 1D is a topside view of an aerosol-generating system in accordance with an embodiment of the present invention;
Figure 2 is a topside view of a tubing segment and a heating coil for an aerosol-generating system in accordance with an embodiment of the present invention;
Figure 3A is a topside view of a tubing segment and a conical heater for an aerosol-generating system in accordance with an embodiment of the present invention;
Figure 3B is schematic illustration for making the conical heater shown in Figure 3A;
Figure 4 is a schematic illustration of an aerosol-generating system in a perspective view in accordance with an embodiment of the present invention; and
Figure 5 is a schematic illustration of an aerosol-generating system in a perspective view and in a cross-sectional view in accordance with an embodiment of the invention.

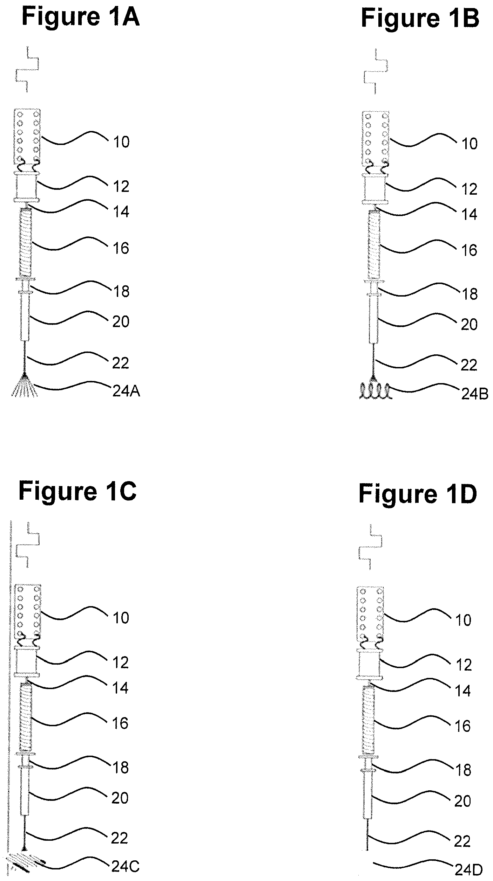

Figure 1A shows an aerosol-generating system comprising electric circuitry 10 that drives a micro stepper motor 12 with a drive shaft 14. Drive shaft 14 is coupled with a lead screw 16 that translates the rotational movement of the drive shaft 14 in response to an electrical pulse of the electric circuitry 10 to an axial movement. The lead screw 16 is connected to a piston 18 that moves a movable wall 26 (not shown in Figure 1A) in capsule 20. Upon a pulse of the electric circuitry 10 to drive the micro stepper motor 12, the available volume in the capsule 20 is reduced by a predetermined amount. The capsule 20 is filled with liquid aerosol-forming substrate. Due to the reduction of volume resulting from pulses, a corresponding amount of liquid aerosol-forming substrate flows into an open-ended nozzle 22 where the liquid aerosol-forming substrate leaves the nozzle via a jet 24A. The jet 24A causes aerosolization of the liquid aerosol-forming substrate.

Figure 1B, 1C, and 1D show aerosol-generating systems with a different handling of the liquid aerosol-forming substrate once the liquid aerosol-forming substrate exits the nozzle 22.

In the embodiment of Figure 1B, a heating coil 24B is arranged downstream of the nozzle 22 to directly heat the liquid aerosol-forming substrate that exits the nozzle 22.

In the embodiment of Figure 1C, a flat heater 24C with a liquid permeable structure is arranged downstream of the nozzle 22 to directly heat the liquid aerosol-forming substrate that exits the nozzle 22.

In the embodiment of Figure 1D, a conical heater 24D is arranged downstream of the nozzle 22 to directly heat the liquid aerosol-forming substrate that exits the nozzle 22.

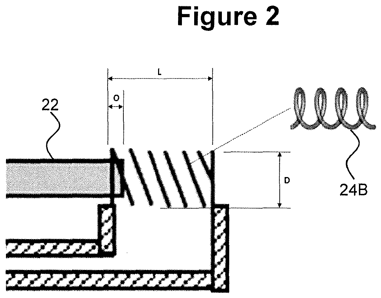

Figure 2 shows a detail of the open ended side of the nozzle 22. A heating coil 24B is mounted onto the open ended side of the nozzle 22 such that the heating coil 24B extends from the nozzle 22 in longitudinal direction. Liquid aerosol-forming substrate exits at the open end of the nozzle 22. The heating coil 24B defines an open-ended internal passage to which the aerosol-forming substrate is delivered by the nozzle 22. The heating coil 24B is in and around the flow of liquid so that the liquid aerosol-forming substrate is directly heated. The heating coil 24B has a length L, a diameter D and an overlap O with the nozzle 22.

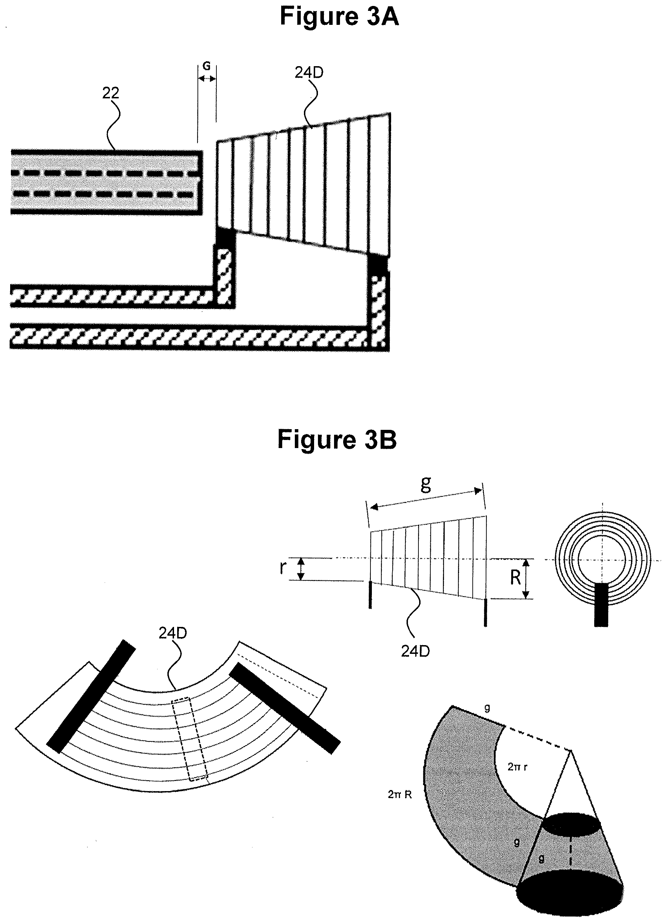

Figure 3A shows a detail of the open ended side of the nozzle 22. A conical heater 24D is mounted downstream the open ended side of the nozzle 22 such that the conical heater 24D extends from the nozzle 22 in longitudinal direction. Liquid aerosol-forming substrate exits at the open end of the nozzle 22. The conical heater 24D defines an internal passage and is in and around the flow of liquid so that the liquid aerosol-forming substrate is directly heated. There is a distance G between the cone end side of the conical heater 24D and the nozzle 22.

Figure 3B is a schematic illustration of making the conical heater 24D from a flat substrate. The conical heater 24D has a slant height g with a radius that increases from a first radius r to a second radius R.

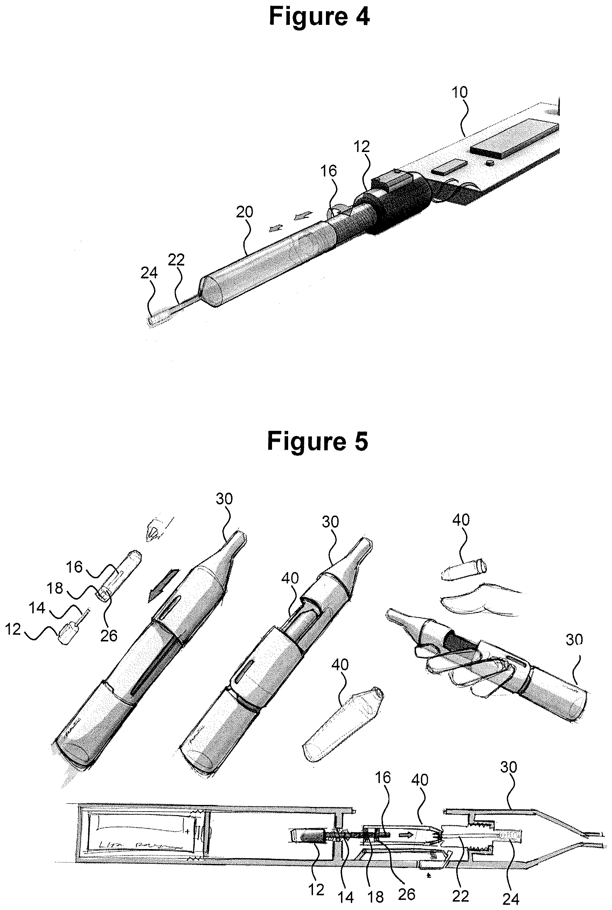

Figure 4 shows the aerosol-generating systems of Figures 1B, 1C, and 1D in a perspective view with a heating element 24 downstream the tubing segment 22.

Figure 5 is a schematic illustration of an aerosol-generating system. The aerosol-generating system comprises a main unit 30 and a separate cartridge 40. The main unit 30 comprises a micro stepper motor 12 with a drive shaft 14. The cartridge 40 comprises a capsule representing the liquid storage portion. The main unit 30 further comprises a tubing segment 22 and a vaporiser 24 receiving liquid aerosol-forming substrate via the tubing segment 22 that extends from the liquid storage portion towards the vapouriser. The vaporiser 24 is configured to heat the liquid aerosol-forming substrate directly after the liquid aerosol-forming substrate exits the tubing segment 22.

Furthermore, the cartridge 40 comprises a lead screw 16 coupled to the drive shaft 14 and a piston 18 that is axially moved by the lead screw 16. The liquid storage portion comprises a movable wall 26 that separates the liquid storage portion from the remaining components inside the capsule of the cartridge.

The cartridge 40 is configured to be received in a cavity within the main unit 30. Cartridge 40 should be replaceable by a user when the aerosol-forming substrate provided in the cartridge 40 is depleted. When inserting a new cartridge 40, a slider at the main unit 30 may be moved to expose the cavity. A new cartridge 40 may be inserted into the exposed cavity. The lead screw 16 of the cartridge 40 comprises an opening for receiving the drive shaft 14 of the micro stepper motor 12. The capsule of the cartridge 40 comprises an outlet for receiving an end of the tubing segment 22.

The main unit 30 is portable and has a size comparable to a conventional cigar or cigarette. The main unit 30 comprises a main body and a mouthpiece portion. The main unit 30 contains a power supply, for example a battery such as a lithium iron phosphate battery, electronic circuitry 10, and a cavity. Electrical connectors are provided at the sides of the main body to provide an electrical connection between the electric circuitry 10 and the battery. The mouthpiece portion comprises a plurality of air inlets and an outlet. In use, a user sucks or puffs on the outlet to draw air from the air inlets, through the mouthpiece portion to the outlet, and thereafter into the mouth or lungs of the user. Internal baffles are provided to force the air flowing through the mouthpiece portion past the cartridge.

The exemplary embodiments described above illustrate but are not limiting. In view of the above discussed exemplary embodiments, other embodiments consistent with the above exemplary embodiments will now be apparent to one of ordinary skill in the art.

## Claims

1. An aerosol-generating system, comprising:
a main unit (30);
a cartridge (40) removably coupled to the main unit;
a power supply provided in the main unit;
a liquid storage portion provided in the cartridge, storing liquid aerosol-forming substrate, wherein the liquid storage portion comprises a movable wall (26) and an outlet;
a vaporiser (24) comprising a heating element having a structure defining an open-ended internal passage;
a pump that delivers liquid aerosol-forming substrate from the outlet of the liquid storage portion to the internal passage of the heating element, where it is heated to a temperature sufficient to volatilize at least a part of the delivered liquid aerosol-forming substrate, the pump comprising:
a micro stepper motor (12) provided in the main unit, with a drive shaft (14) that is configured to rotate for a predetermined amount upon performing one step of the micro stepper motor;
a piston (18) connected to the movable wall; and
a lead screw (16) connecting the drive shaft to the piston and configured to translate a rotation of the drive shaft into an axial movement of the piston and a corresponding axial movement of the movable wall;
wherein the vaporiser heats the delivered liquid aerosol-forming substrate in the internal passage to a temperature sufficient to volatilize at least a part of the delivered liquid aerosol-forming substrate.

2. An aerosol-generating system according to claim 1, wherein the liquid storage portion is configured such that the axial movement of the movable wall (26) towards the liquid storage portion causes a reduction of the volume of the liquid storage portion so as to deliver a determined amount of liquid aerosol-forming substrate from the outlet of the liquid storage portion to the internal passage of the heating element upon performing one step of the micro stepper motor (12).

3. An aerosol-generating system according to claim 1 or claim 2, wherein the micro stepper motor (12) is further configured to perform a step in reverse direction, thereby increasing the volume of the liquid storage portion.

4. An aerosol-generating system according to any of claims 1 to 3, wherein the movable wall (26) is configured to contain the liquid aerosol-forming substrate in the liquid storage portion so that the micro stepper motor (12) and the piston (18) are not in contact with the liquid aerosol-forming substrate.

5. An aerosol-generating system according to any of claims 1 to 4, wherein the system further comprises a chamber into which the liquid aerosol-forming substrate is delivered, and wherein the heating element is arranged inside the chamber downstream of the outlet of the liquid storage portion.

6. An aerosol-generating system according to any of claims 1 to 5, wherein the system further comprises a tubing segment (22) through which the liquid aerosol-forming substrate is delivered from the liquid storage portion to the vaporiser.

7. An aerosol-generating system according to claim 6, wherein the vaporiser is arranged downstream of an open end of the tubing segment (22).

8. An aerosol-generating system according to claim 6 or 7, wherein the tubing segment (22) comprises a capillary tube.

9. An aerosol-generating system according to claim 6 or claim 7 or claim 8, wherein the vaporiser comprises a heating coil (24B) extending around the tubing segment (22).

10. An aerosol-generating system according to any one of claims 6 to 9, wherein the vaporiser comprises a heating coil (24B) extending from the tubing segment (22) in longitudinal direction.

11. An aerosol-generating system according to claim 6 or claim 7 or claim 8, wherein the vaporiser comprises a conical heater (24D) extending from the tubing segment (22) in longitudinal direction.

12. An aerosol-generating system according to any of claims 1 to 11, wherein the liquid storage portion comprises a one-way valve connected to the outlet of the liquid storage portion.

13. An aerosol-generating system according to any of claims 1 to 12, wherein the flow rate of the liquid aerosol-forming substrate delivered through the outlet of the liquid storage portion is within 0.5 to 2 microlitres per second.

## Patentansprüche

1. Aerosolerzeugungssystem, aufweisend:
eine Haupteinheit (30);
eine Patrone (40), die mit der Haupteinheit lösbar gekoppelt ist;
eine Energieversorgung, die in der Haupteinheit vorgesehen ist;
ein in der Patrone vorgesehener Flüssigspeicherteil, der ein flüssiges aerosolbildendes Substrat speichert, wobei der Flüssigspeicherteil eine bewegliche Wand (26) und einen Auslass aufweist;
einen Zerstäuber (24), aufweisend ein Heizelement mit einer Struktur, die einen offenen inneren Durchgang definiert;
eine Pumpe, die flüssiges aerosolbildendes Substrat von dem Auslass des Flüssigspeicherteils an den inneren Durchgang des Heizelements liefert, wo es auf eine Temperatur erwärmt wird, die ausreichend ist, um zumindest einen Teil des gelieferten flüssigen aerosolbildenden Substrats zu verflüchtigen, die Pumpe Folgendes aufweisend:
einen in der Haupteinheit vorgesehenen Mikroschrittmotor (12) mit einer Antriebswelle (14), die so ausgelegt ist, dass sie sich bei Ausführung eines Schritts des Mikroschrittmotors um einen vorbestimmten Betrag dreht;
einen Kolben (18), der mit der beweglichen Wand verbunden ist; und
eine Leitspindel (16), die die Antriebswelle mit dem Kolben verbindet und so ausgelegt ist, dass sie eine Drehung der Antriebswelle in eine axiale Bewegung des Kolbens und eine entsprechende axiale Bewegung der beweglichen Wand übersetzt;
wobei der Zerstäuber das gelieferte flüssige aerosolbildende Substrat in dem inneren Durchgang auf eine Temperatur erwärmt, die ausreichend ist, um zumindest einen Teil des gelieferten flüssigen aerosolbildenden Substrats zu verflüchtigen.

2. Aerosolerzeugungssystem nach Anspruch 1, wobei der Flüssigspeicherteil so ausgelegt ist, dass die axiale Bewegung der beweglichen Wand (26) in Richtung des Flüssigspeicherteils eine Verringerung des Volumens des Flüssigspeicherteils bewirkt, sodass bei Ausführung eines Schritts des Mikroschrittmotors (12) eine bestimmte Menge des flüssigen aerosolbildenden Substrats aus dem Auslass des Flüssigspeicherteils an den inneren Durchgang des Heizelements geliefert wird.

3. Aerosolerzeugungssystem nach Anspruch 1 oder Anspruch 2, wobei der Mikroschrittmotor (12) ferner ausgelegt ist, um einen Schritt in umgekehrter Richtung auszuführen, wodurch das Volumen des Flüssigspeicherteils erhöht wird.

4. Aerosolerzeugungssystem nach einem der Ansprüche 1 bis 3, wobei die bewegliche Wand (26) so ausgelegt ist, dass sie das flüssige aerosolbildende Substrat in dem Flüssigspeicherteil enthält, sodass der Mikroschrittmotor (12) und der Kolben (18) nicht in Kontakt mit dem flüssigen aerosolbildenden Substrat sind.

5. Aerosolerzeugungssystem nach einem der Ansprüche 1 bis 4, wobei das System ferner eine Kammer aufweist, in die das flüssige aerosolbildende Substrat geliefert wird, und wobei das Heizelement innerhalb der Kammer einem Auslass des Flüssigspeicherteils nachgeschaltet angeordnet ist.

6. Aerosolerzeugungssystem nach einem der Ansprüche 1 bis 5, wobei das System ferner ein Rohrleitungssegment (22) aufweist, durch das das flüssige aerosolbildende Substrat von dem Flüssigspeicherteil an den Zerstäuber geliefert wird.

7. Aerosolerzeugungssystem nach Anspruch 6, wobei der Zerstäuber einem offenen Ende des Rohrleitungssegments (22) nachgeschaltet angeordnet ist.

8. Aerosolerzeugungssystem nach Anspruch 6 oder 7, wobei das Rohrleitungssegment (22) ein Kapillarrohr aufweist.

9. Aerosolerzeugungssystem nach Anspruch 6 oder Anspruch 7 oder Anspruch 8, wobei der Zerstäuber eine Heizspule (24B) aufweist, die sich um das Rohrleitungssegment (22) erstreckt.

10. Aerosolerzeugungssystem nach einem der Ansprüche 6 bis 9, wobei der Zerstäuber eine Heizspule (24B) aufweist, die sich von dem Rohrleitungssegment (22) in Längsrichtung erstreckt.

11. Aerosolerzeugungssystem nach Anspruch 6 oder Anspruch 7 oder Anspruch 8, wobei der Zerstäuber eine kegelförmige Heizvorrichtung (24D) aufweist, die sich von dem Rohrleitungssegment (22) in Längsrichtung erstreckt.

12. Aerosolerzeugungssystem nach einem der Ansprüche 1 bis 11, wobei der Flüssigspeicherteil ein Einwegventil aufweist, das mit dem Auslass des Flüssigspeicherteils verbunden ist.

13. Aerosolerzeugungssystem nach einem der Ansprüche 1 bis 12, wobei die Strömungsgeschwindigkeit des durch den Auslass des Flüssigspeicherteils gelieferten, flüssigen aerosolbildenden Substrats innerhalb von 0,5 bis 2 Mikroliter pro Sekunde liegt.

## Revendications

1. Système de génération d'aérosol, comprenant :
une unité principale (30) ;
une cartouche (40) couplée d'une manière amovible à l'unité principale ;
une alimentation électrique prévue dans l'unité principale ;
une portion de stockage de liquide prévue dans la cartouche, stockant un substrat formant aérosol liquide, dans lequel la portion de stockage de liquide comprend une paroi mobile (26) et une sortie ;
un vaporisateur (24) comprenant un élément de chauffage ayant une structure définissant un passage interne ouvert ;
une pompe qui fournit un substrat formant aérosol liquide depuis la sortie de la portion de stockage de liquide vers le passage interne de l'élément de chauffage, où il est chauffé jusqu'à une température suffisante pour volatiliser au moins une partie du substrat formant aérosol liquide fourni, la pompe comprenant :
un micromoteur pas à pas (12) prévu dans l'unité principale, avec un arbre d'entraînement (14) qui est configuré pour tourner d'une quantité prédéterminée lors de l'exécution d'un pas du micromoteur pas à pas ;
un piston (18) raccordé à la paroi mobile ; et
une tige filetée (16) raccordant l'arbre d'entraînement au piston et configurée pour translater une rotation de l'arbre d'entraînement en un mouvement axial du piston et un mouvement axial correspondant de la paroi mobile ;
dans lequel le vaporisateur chauffe le substrat formant aérosol liquide fourni dans le passage interne jusqu'à une température suffisante pour volatiliser au moins une partie du substrat formant aérosol liquide fourni.

2. Système de génération d'aérosol selon la revendication 1, dans lequel la portion de stockage de liquide est configurée de telle sorte que le mouvement axial de la paroi mobile (26) vers la portion de stockage de liquide provoque une réduction du volume de la portion de stockage de liquide de manière à fournir une quantité déterminée de substrat formant aérosol liquide depuis la sortie de la portion de stockage de liquide vers le passage interne de l'élément de chauffage lors de l'exécution d'un pas du micromoteur pas à pas (12).

3. Système de génération d'aérosol selon la revendication 1 ou la revendication 2, dans lequel le micromoteur pas à pas (12) est en outre configuré pour exécuter un pas dans le sens inverse, augmentant ainsi le volume de la portion de stockage de liquide.

4. Système de génération d'aérosol selon l'une quelconque des revendications 1 à 3, dans lequel la paroi mobile (26) est configurée pour contenir le substrat formant aérosol liquide dans la portion de stockage de liquide de sorte que le micromoteur pas à pas (12) et le piston (18) ne sont pas en contact avec le substrat formant aérosol liquide.

5. Système de génération d'aérosol selon l'une quelconque des revendications 1 à 4, dans lequel le système comprend en outre une chambre dans laquelle le substrat formant aérosol liquide est fourni, et dans lequel l'élément de chauffage est disposé à l'intérieur de la chambre en aval de la sortie de la portion de stockage de liquide.

6. Système de génération d'aérosol selon l'une quelconque des revendications 1 à 5, dans lequel le système comprend en outre un segment de tubulure (22) à travers lequel le substrat formant aérosol liquide est fourni depuis la portion de stockage de liquide vers le vaporisateur.

7. Système de génération d'aérosol selon la revendication 6, dans lequel le vaporisateur est disposé en aval d'une extrémité ouverte du segment de tubulure (22).

8. Système de génération d'aérosol selon la revendication 6 ou 7, dans lequel le segment de tubulure (22) comprend un tube capillaire.

9. Système de génération d'aérosol selon la revendication 6 ou la revendication 7 ou la revendication 8, dans lequel le vaporisateur comprend une résistance chauffante (24B) s'étendant autour du segment de tubulure (22).

10. Système de génération d'aérosol selon l'une quelconque des revendications 6 à 9, dans lequel le vaporisateur comprend une résistance chauffante (24B) s'étendant depuis le segment de tubulure (22) dans la direction longitudinale.

11. Système de génération d'aérosol selon la revendication 6 ou la revendication 7 ou la revendication 8, dans lequel le vaporisateur comprend un dispositif de chauffage conique (24D) s'étendant depuis le segment de tubulure (22) dans la direction longitudinale.

12. Système de génération d'aérosol selon l'une quelconque des revendications 1 à 11, dans lequel la portion de stockage de liquide comprend un clapet unidirectionnel raccordé à la sortie de la portion de stockage de liquide.

13. Système de génération d'aérosol selon l'une quelconque des revendications 1 à 12, dans lequel le débit du substrat formant aérosol liquide fourni à travers la sortie de la portion de stockage de liquide est de 0,5 et 2 microlitres par seconde.
